# EUROPEAN PATENT APPLICATION

(11) **EP 3 695 798 A1**
(43) Date of publication of application: **19.08.2020**
(21) Application number: 19157020.9
(22) Date of filing: 13.02.2019
(51) Int. Cl.: A61B 18/14

(54) **AN ABLATION PROBE**

(71) Applicant: National University of Ireland Galway, Galway (IE)
(72) Inventor: EATON-EVANS, Jimmy, Galway (IE); RUVIO, Giuseppe, Galway (IE); BOUCHIER-HAYES, Jonathan, Galway (IE); O'HALLORAN, Martin, Galway (IE); BRUZZI, Mark, Galway (IE)
(74) Representative: Barker Brettell LLP

(57) **Abstract**

An ablation probe (100; 200) comprising: an applicator (102; 202) arranged to apply radiation to heat surrounding tissue; a feeding cable (104; 204) arranged to supply electromagnetic energy to the applicator; a coolant flow path (106, 108) forming a coolant supply circuit; a tubular member (112; 212) housing at least part of the feeding cable (104; 204), wherein a part of the coolant flow path is defined by a space between the feeding cable and the tubular member; and a coupling body (114). The coupling body comprises: a cavity (116) in which the applicator (102; 204) is at least partly encapsulated; a coupling interface (118) at which the coupling body (114) is coupled to the tubular member; and a pointed distal tip (114a) adapted for piercing tissue.

## Description

This application relates to an ablation probe. In particular, this application relates to an ablation probe that may be used to generate heat within tissue to destroy tissue growths.

Thermal ablation can be used to destroy tissue growths within the body which can be malignant. Current ablation systems use applicators that deliver Radio Frequency (RF) energy (or microwave energy) to the tissue surrounding the applicator tip. This causes localised heating and destruction of the malignant cells. These applicators may be designed for percutaneous delivery and are therefore relatively short in length and large in diameter. However, many disease locations cannot be safely or easily accessed percutaneously. For example, the location of the pancreas behind the liver makes it difficult to access percutaneously. Similarly, access to the lung through the chest wall can cause a pneumothorax. Large diameter applicators may also cause undesired tissue damage during insertion. This limits the range of indications where thermal ablation therapy can be successfully delivered using existing percutaneous applicators.

Various sites within the human body can be accessed by navigating through a natural orifice. For example the periphery of the lung can be accessed using lung navigation systems, or similar devices such as an endoscope, that guide a working channel through the airway network to a target. This enables therapies to be delivered through the device working channel to diagnose and treat disease. Microwave ablation can be delivered via these systems. However, a long and flexible ablation catheter is required that is capable of delivering sufficient power to its radiating tip. Known microwave systems use coaxial cable to deliver power, with larger diameter cables used to generate fewer electrical losses than smaller gauge cables. However, small diameter cables improve flexibility, reduce insertion profile and require less force to straighten if plastically deformed during delivery. It is not practical to run a small cable (e.g. diameter < 0.7 mm) over the length necessary to reach many target sites (e.g. >1 m for lung) because the electrical losses would be too great, and may result in excessive heating effects and insufficient power delivery (resulting in excessively long treatment times).

In the applicant's previous European application No. EP17164403.2 filed on 31 March 2017, a microwave ablation probe having a feeding cable arranged to supply electromagnetic energy to an applicator was disclosed. The feeding cable comprises a proximal portion and a distal portion having different cross section sizes to each other. A connector is also provided to mechanically and electrically splice the distal portion of the feeding cable to the proximal portion. EP17164403.2 also disclosed the use of a deformable member which provides a coolant path through which coolant is able to flow.

Improvements to known ablation probes such as this are desired to allow efficient use with delivery devices such as endoscopes or Electromagnetic Navigation Bronchoscopy (ENB) Systems. A suitable level of flexibility must be provided so that a tortuous route through anatomy can be followed (e.g. bend radius of less than 10mm). The ablation probe must also be compact in size so that it can be used with a narrow working channel (e.g. 2 mm diameter). Reducing the size of the ablation probe can lead to problems in maintaining mechanical strength so that the device does not fail during delivery or therapy. A secure connection between components is therefore desired, which can be difficult to achieve where components are formed from different, incompatible, materials. Moreover, a simple geometry is desired to help aid manufacture and allow volume production and reliability. The overall assembly must be flexible enough for delivery through a torturous path to reach the disease location.

In a first aspect, the present application provides an ablation probe, comprising any one or more of:
an applicator arranged to apply radiation to heat surrounding tissue;
a feeding cable arranged to supply electromagnetic energy to the applicator;
a coolant flow path forming a coolant supply circuit;
a tubular member housing at least part of the feeding cable, wherein a part (e.g. a first part) of the coolant flow path is defined by a space between the feeding cable and the tubular member;
and a coupling body, the coupling body comprising:
   a cavity in which the applicator is at least partly encapsulated;
   a coupling interface at which the coupling body is coupled to the tubular member;
   and a pointed distal tip adapted for piercing tissue.

The coupling body acts as a single component with which the applicator and tubular member are secured together. By encapsulating the applicator within the coupling body a secure mechanical connection to the applicator can be made with less reliance on adhesives or machining the applicator (which may be formed from a dielectric) into a complex shape. The material of the coupling body may be different from that of the applicator and so more compatible for bonding to other parts of the ablation probe during assembly.

Optionally, the ablation probe may further comprise a deformable member surrounding at least part of the tubular member, the deformable member being arranged to move between an insertion configuration and a deployed configuration, and wherein a part (e.g. a second part) of the coolant path is defined between the deformable member and the tubular member when the deformable member is in the deployed configuration, and wherein the coupling body comprises a coupling interface at which the coupling body is coupled to the deformable member.

Optionally, the coupling body may be formed at least partly from a plastics material. This may aid manufacture; have desirable flexibility and toughness; and provide a suitable material with which to couple the tubular member and/or deformable member.

The material with which the coupling body is formed may have properties as defined in any one or a combination of statements a) to f) below:
a) Optionally, the coupling body may be formed at least partly from a material having a Charpy notched impact strength in the range between 1 and 50 kJ/m².
b) Optionally, the coupling body may be formed at least partly from a material having a Rockwell Harness (m-Scale) in the range between 10 and 50.
c) Optionally, the coupling body may be formed at least partly from a material having a flexural modulus in the range between 1-50 GPa.
d) Optionally, the coupling body may be formed at least partly from a material having a heat deflection temperature in the range between 80 and 400 degrees Celsius at 1.8 MPa.
e) Optionally the coupling body may be formed at least partly from a transparent material.
f) Optionally, the deformable member and at least a portion of the coupling body forming the coupling interface at which the coupling body is coupled to the deformable member are both formed from materials compatible for thermal welding. This may include materials that have equal or similar melting points (e.g. melting points within 100 degrees or within 50 degrees of each other).

Optionally, the coupling body may be formed at least partly from and one of: polycarbonate (PC), Polyetheretherketone (PEEK), nylon (e.g. nylon 6), glass reinforced nylon (e.g. glass reinforced nylon 6), Liquid Crystal Polymer (LCP), polystyrene, polyethylene terephthalate (PET) or a thermoset material (e.g. polyimide).

Optionally, the coupling body may be formed from nylon or glass reinforced nylon and the tubular member is formed at least partly from a polymeric material such as Nylon, Pebax or PEEK. This may aid thermal welding of the coupling body and tubular member.

Optionally, the coupling body may be formed from nylon or glass reinforced nylon and the deformable member is formed at least partly from nylon. This may aid thermal welding of the coupling body and deformable member.

Optionally, the applicator may comprise an antenna body and an antenna conductor, wherein the antenna body comprises an outer surface having a channel in which the antenna conductor is received. The antenna conductor may be formed from an elongate wire component, with the channel following a path around and/or along the outer surface having a shape corresponding (e.g. the same as) to the shape of the path followed by the antenna conductor. The channel is therefore adapted to maintain the shape of the antenna conductor.

Optionally, the channel may extend along a helical path around a central axis of the antenna body.

Optionally, the feeding cable may comprise an inner conductor, an outer conductor and a dielectric material between them, and wherein the antenna conductor is formed from part of the inner conductor of the feeding cable.

Optionally part of the length of the antenna conductor extends in a distal direction from a distal end of the outer conductor and within an axial through hole formed in the antenna body.

Optionally, a potting compound or adhesive is provided between the applicator and the coupling body.

Optionally, the coupling body is insert moulded around the applicator.

Optionally, the coupling interface at which the coupling body is coupled to the tubular member may comprise an overlapping portion of the coupling body that extends within or around the tubular member so that they overlap one another.

Optionally, the overlapping portion forms a reduced thickness portion of the coupling body overlapping the tubular member. This may help to enhance flexibility or strain relief at this joint.

Optionally, the pointed distal tip of the coupling body may be provided by a separate tip member coupled to the distal end of the coupling body. The tip member may be coupled via an interlocking profile (e.g. a socket) provided on the distal end of the coupling body. The tip member may be formed from a metal, metal alloy or ceramic material.

Optionally, the ablation probe further comprises a sheath member movable between a first position in which it surrounds the pointed tip of the coupling body and a second position in which the pointed tip is uncovered.

Optionally, the sheath member may extend part way along the length of the ablation probe between the distal and proximal ends of the ablation probe, and wherein the ablation probe comprises one or more control lines connected to the sheath member, the control lines extending along the length of the ablation probe between the sheath member and a position at or near the proximal end of the ablation probe.

Optionally the one or more control lines may be connected at or near a distal end of the sheath member.

Optionally, the sheath member is formed from a tube having a reinforcing ring at its distal end, wherein the one or more control lines are connected to the reinforcing ring.

Optionally, the ablation probe is slidably coupled to a handle provided at or near its proximal end, and wherein the one or more control lines are connected between the handle and the sheath member, and the control lines are arranged to restrict the range of movement of the sheath in a proximal direction away from the handle.

Optionally, the ablation probe comprises an outer catheter tube in which part of the coolant flow path is contained, and wherein the one or more control lines extend within a channel or lumen formed in the outer catheter tube.

Optionally, the sheath member forms a friction fit with the body of the ablation probe around which it extends and/or comprises a biasing member arranged to bias the sheath member in a distal direction.

Optionally, the sheath member may comprise a covering member arranged to cover the pointed tip when the sheath member is in the first position, the covering member being pierced by the pointed tip when the sheath member is moved from the first position to the second position to expose the pointed tip. The covering member may be a membrane extending across the distal end of the sheath member.

In a second aspect, the present application provides an ablation probe, comprising any one or more of:
an applicator arranged to apply radiation to heat surrounding tissue;
a feeding cable arranged to supply electromagnetic energy to the applicator;
a pointed distal tip adapted for piercing tissue;
a sheath member movable between a first position in which it surrounds the pointed tip and a second position in which the pointed tip is uncovered, the sheath member extending part way along the length of the ablation probe the distal and proximal ends of the ablation probe; and one or more control lines connected to the sheath member, the control lines extending along the length of the ablation probe between the sheath member and a position at or near the proximal end of the ablation probe.

By coupling the sheath member via one or more control lines its position can be controlled without its length running along the full length of the ablation probe back to the proximal end. This saves space taken up by the device in the working channel of a delivery device with which it is used.

Optionally the one or more control lines may be connected at or near a distal end of the sheath member.

Optionally, the sheath member is formed from a tube having a reinforcing ring at its distal end, wherein the one or more control lines are connected to the reinforcing ring.

Optionally, the ablation probe is slidably coupled to a handle provided at or near its proximal end, and wherein the one or more control lines are connected between the handle and the sheath member, and the control lines are arranged to restrict the range of movement of the sheath in a proximal direction away from the handle.

Optionally, the ablation probe comprises an outer catheter tube in which part of the coolant flow path is contained, and wherein the one or more control lines extend within a channel or lumen formed in the outer catheter tube.

Optionally, the sheath member forms a friction fit with the body of the ablation probe around which it extends and/or comprises a biasing member arranged to bias the sheath member in a distal direction.

Optionally, the sheath member may comprise a covering member arranged to cover the pointed tip when the sheath member is in the first position, the covering member being pierced by the pointed tip when the sheath member is moved from the first position to the second position to expose the pointed tip. The covering member may be a membrane extending across the distal end of the sheath member.

Any of the features disclosed in the statements above (or elsewhere herein) in connection with the first aspect may be provided in combination with the second aspect. Any of the features disclosed in the statements above (or elsewhere herein) in connection with the second aspect may be provided in combination with the first aspect.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality.

Embodiments of the invention will now be described, by way of example only, with reference to the accompanying drawings, in which:
**Figure 1** shows a side view of an ablation probe according to an embodiment;
**Figure 2** shows an exploded view of part of the ablation probe shown in Figure 1;
**Figure 3** shows a cross section view of part of the ablation probe shown in Figure 1;
**Figure 4** shows another cross section view of part of the ablation probe shown in Figure 1;
**Figure 5** shows a close up view of the applicator of the ablation probe shown in Figure 1;
**Figure 6** shows a close up cross section view of the boundary between a needle portion and a catheter portion of the ablation probe shown in Figure 1;
**Figures 7a and 7b** show side views of an ablation probe having a sheath member according to another embodiment;
**Figure 8** shows a close up cross section view of the sheath member of the ablation probe shown in Figures 7a and 7b;
**Figures 9a and 9b** show side views of the ablation probe shown in Figure 7a being moved relative to a handle to which the ablation probe is coupled; and
**Figure 10** shows an ablation probe having a sheath according to another embodiment.

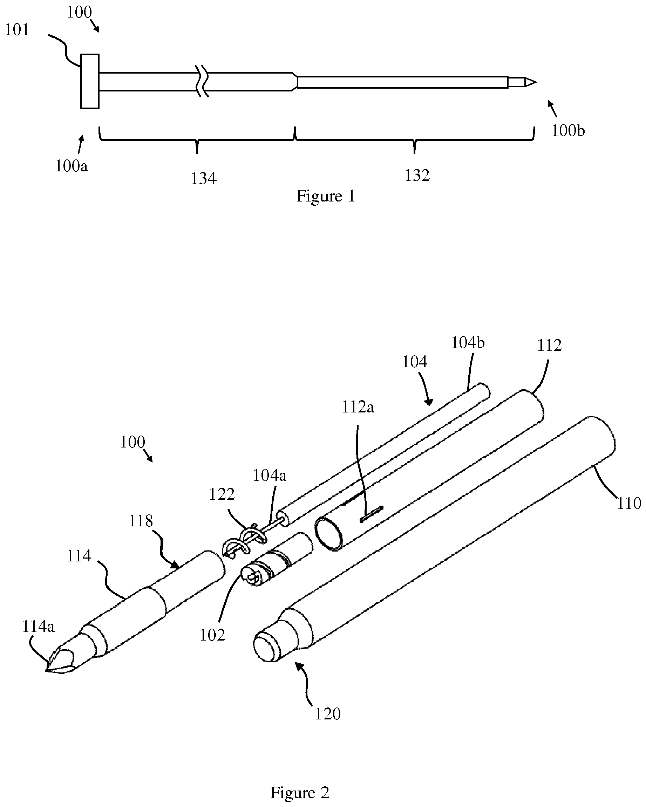

An ablation probe 100 according to an embodiment is shown schematically in Figure 1. The ablation probe 100 of the present disclosure may be suitable for insertion into the body to reach a desired treatment site, such as a malignant tissue growth. In order to reach a desired treatment site, the ablation probe may be suitable for insertion through the working channel of an internal anatomy access device. By internal anatomy access device we mean any device which may be placed within the anatomy of a patient, the device having a working channel for insertion of instruments to a desired location within the body. The internal anatomy device may be an intraluminal delivery device arranged to be delivered along an anatomical lumen of the patient (e.g. the trachea and the pathways of the bronchi in the lungs or the oesophagus). The ablation probe 100 may, for example, be used endoscopically or using an ENB system in order to reach a variety of disease locations within the body. The ablation probe may therefore have an overall flexibility such that it can be inserted through the working channel of the endoscope. In other embodiments, the ablation probe may be used with other types of intraluminal delivery device such as specific types of endoscope (e.g. a bronchoscope) or a navigation system such as a lung navigation system (e.g. an ENB system). In other examples, the ablation probe 100 may also be used percutaneously, or using any other suitable technique, e.g. inserted through an existing aperture of the body. For percutaneous use, the ablation probe may be generally rigid so that it can be inserted.

The ablation probe extends between a proximal end 100a and a distal end 100b. The terms "distal" and "proximal" are taken relative to the user operating the ablation probe and the treatment site when the ablation probe is positioned for use - the distal end 100b of the ablation probe 100 is that closest to the treatment site and the proximal end 100a is that closest to the user. A handle 101 is provided at the proximal end of the ablation probe 100 so that it can be manipulated and positioned by the user. The distal end 100b may be fed through the working channel of an endoscope or similar device to reach a target ablation site.

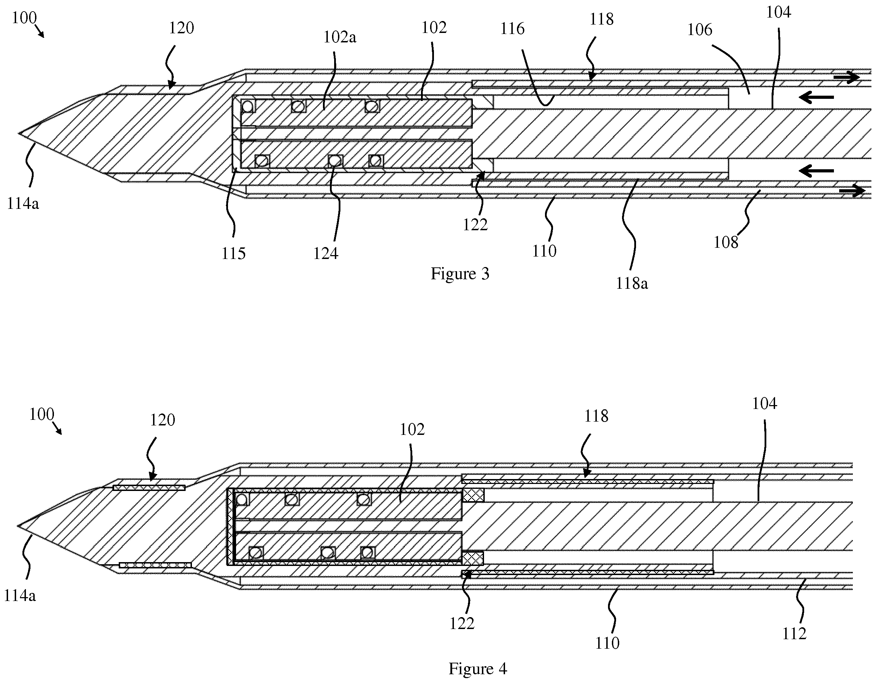

An exploded and a sectional view of the distal end of the ablation probe 100 are shown in Figures 2 and 3 respectively.

The ablation probe 100 comprises an applicator 102 arranged to apply radiation to heat surrounding tissue. The applied radiation may be adapted to cause localised heating and destruction of malignant cells around or near to the applicator 102. The applicator 102 may be arranged to apply any suitable form of radiation to surrounding tissue such that the desired heating is caused. The applicator 102 may, for example, be arranged to emit microwave or RF radiation, or may emit any other suitable radiation to cause heating. The applicator 102 is arranged at or near a distal end of the ablation probe 100 so that it can be positioned in a desired position relative to the tissue to be treated. The applicator 102 may be formed from a ceramic material with suitable dielectric properties (for example, zirconia) according to the energy it is arranged to apply.

The ablation probe 100 further comprises a feeding cable 104 which is arranged to supply electromagnetic energy to the applicator 102. Only part of the length of the feeding cable is shown in Figures 2 and 3. The feeding cable 104 may be any elongate member suitable for supplying electromagnetic energy to the applicator (e.g. a conductor). The feeding cable 104 may run along at least part or all of the length of the ablation probe 100 to deliver a supply of energy to the applicator 102. In the described embodiment, a distal end of the feeding cable 104 is coupled to a proximal end of the applicator 102 and a proximal end of the feeding cable 104 (not shown in Figures 2 and 3) is coupled to a generation means (also not shown in the Figures) suitable for generating the desired signal to supply energy to the applicator 102.

The ablation probe further comprises a coolant circuit flow path forming a coolant supply circuit. The flow of coolant is shown by arrows in Figure 3. The coolant circuit flow path comprises a first coolant path 106. In the described embodiment, the first coolant path is a coolant delivery path via which coolant is able to flow in a direction towards the applicator 102. For example, the coolant delivery path 106 may deliver a flow of coolant towards the distal end of the ablation probe 100 from a coolant supply means (not shown in the Figures) coupled to the coolant delivery path 106 at the proximal end of the ablation probe 100. The flow of coolant may help control the temperature of the ablation probe 100 during use. This may allow energy to be delivered to the surrounding tissue for an extended period of time without the ablation probe 100 overheating and being damaged, or causing injury to healthy tissue. The coolant may be a fluid, and may be water, saline solution, a cryogenic gas or any other suitable coolant known in the art.

The coolant circuit flow path further comprises a second coolant path 108. In the described embodiment, the second coolant path 108 is a coolant return path via which coolant can return from the applicator. The coolant return path 108 may therefore return the supply of coolant from the distal end of the ablation probe 100 to the proximal end.

The ablation probe 100 further comprises a deformable member 110 which is arranged to move between an insertion configuration in which insertion of the ablation probe 100 is facilitated and a deployed configuration (shown in Figure 3). When in the deployed configuration, the coolant return path 108 is provided by the deformable member 110. In some embodiments, no coolant return path may be provided when the deformable member is in the insertion configuration. This may allow the profile of the ablation probe to be minimised. In other embodiments, the return path may not be completely absent when the deformable member is in the insertion configuration. The insertion configuration therefore provides a configuration in which the ablation probe 100 may be suitable for delivery to the desired location within the body. The insertion configuration may, for example, correspond to a suitable size and/or shape adapted to allow insertion with reduced risk of undesired tissue damage. When in the insertion configuration, the ablation probe 100 may, for example, have a low profile (e.g. small cross sectional size) for ease of insertion through tissue without causing injury or insertion through the working channel of an endoscope.

In other embodiments, the first coolant path 106 may act as a coolant return path. In this embodiment, the first coolant path 106 is arranged to carry a flow of coolant away from the applicator. In this embodiment, the second coolant path 108 may act as a coolant delivery path arranged to carry a flow of coolant towards the applicator. A combination of the first and second coolant paths may therefore form a coolant circuit arranged to deliver a flow of coolant towards and away from the applicator, where the coolant can flow in either direction along each of the first and second coolant paths.

The ablation probe 100 may be delivered to the desired location whilst the deformable member 110 is in the insertion configuration. Once at the desired location, the deformable member 110 may be moved to the deployed configuration to allow flow of the coolant away from the applicator 102. The coolant can then flow via the coolant delivery and return paths to cool the ablation probe 100 during use. The deformable member 110 therefore is able to provide an insertion configuration suitable for delivery to the ablation site when the coolant flow is not required. Once the ablation probe is in position, the deformable member 110 may be moved to a configuration suitable to provide a flow of coolant as required during delivery of energy from the applicator 102. When in the deformable member is in the insertion configuration the overall diameter of the ablation probe may be between about 13 to about 25 gauge (approximately 2.5 to 0.5 mm). This may allow easy insertion.

As can be seen in Figures 2 and 3, the coolant return path 110 may be provided only by the deformable member along at least a portion of a length of the ablation probe 100. For example, along at least part of the length of the ablation probe 100, no other channels or conduits to carry returning coolant may be provided in addition to the coolant return path 108 formed by the deformable member 110. This may allow the ablation probe 100 to have a small cross sectional size when the deformable member is in the insertion configuration.

In some embodiments, the deformable member may not be provided. In such embodiments, a non-deformable tubular member or similar suitable component may be provided in which to contain the coolant return path 110.

The ablation probe further comprises a tubular member 112 (e.g. a hypotube, or braid / coil reinforced tubing) arranged to house at least part of the length of the feeding cable 104. The tubular member 112 may be formed from a metal material which has sufficient rigidity to allow the ablation probe to be inserted into tissue. In other embodiments, the tubular member 112 may be formed from any other suitable material, and may be formed from a superelastic material, for example Nitinol.

In other embodiments, the tubular member 112 may be formed form an elastic material (and not specifically a superelastic material). By forming the tube from an elastic (or superelastic) material it may withstand permanent deformation after being delivered through the tortuous path of a working channel. As the ablation probe extends from the working channel it may consequently follow a straight path, rather than following a curved path caused by the material being deformed by the shape of the working channel. This may help to more easily guide the distal tip of the ablation probe to the desired position.

In the presently described embodiment, the coolant delivery path 106 is provided by a channel formed between the feeding cable 104 and inside wall of the tubular member 112. For example, clearance between the feeding cable 104 and the inside wall of the tubular member 112 may provide space for coolant to flow. In other embodiments, slots may be cut into the inside wall of the tubular member 112 to provide a space through which coolant can flow. The amount of clearance may be specified to ensure an adequate flow of cooling is achieved while maximising the power carrying capacity of the feeding cable.

The tubular member 112 may comprise one or more holes 112a through which coolant is able to flow between the first part of the coolant circuit flow path within the deformable member 110 and the second part of the coolant circuit flow path within the tubular member. The holes 112a may be located at or near the distal end of the tubular member to provide a flow of coolant at or near to the applicator 102.

The deformable member 110 is formed by an inflatable member arranged to move between a deflated configuration when the deformable member 110 is in the insertion configuration and an inflated configuration when the deformable member 110 is in the deployed configuration. The inflatable member may thus form a balloon which may be inflated by the flow of coolant (e.g. the inflatable member may inflate due to the pressure of the coolant). In the described embodiment, the inflatable member has an inside diameter that matches the outside diameter of the tubular member 112 which is surrounds. The inflating member may inflate to a larger diameter when the cooling system is pressurised. This may therefore form a conduit for the cooling fluid to return from the applicator 102. When moving to the inflated configuration, some, or all, of the inflating member may change shape (e.g. expand) to allow space for the coolant to flow. When the inflation member is deflated, the insertion profile of the ablation probe 100 may be reduced (e.g. minimised) to aid delivery to the target ablation site. When the ablation therapy has been delivered, the inflation member may be deflated so that it returns to its original diameter to facilitate removal.

The ablation probe 100 further comprises a coupling body 114. The coupling body is adapted to structurally couple the applicator, tubular member and deformable member (where provided) together. The coupling body 114 comprises a cavity 116 in which the applicator 104 is at least partly encapsulated. In order to house the applicator, the coupling body 114 may comprise a hole (e.g. an axial bore hole) extending from its proximal end. The hole may be sized to receive the applicator 102. In the present embodiment, all of the applicator 102 is inserted into the cavity 116 so that it is contained within the coupling body 114. The feeding cable 104 extends along the length of the cavity 116, and out of its open end so as to allow a connection to the applicator 102.

The tubular member 112 may be assembled over (or into) the coupling body 114 to align with, and/or be proximal of, the applicator feedpoint. The applicator feedpoint is the point at which the feeding cable connects to the applicator (i.e. where the inner conductor of the feeding cable is inserted into the applicator as will be described later). The applicator 102 may include a counter bored recess, proximally to accommodate the cable outer conductor and ensure an overlap is formed between the applicator 102 and the tubular member 112. The counter bored recess may form the tubular member coupling interface and feeding cable interface as described later.

In some embodiments, the coupling body 114 may be moulded around (e.g. using an insert moulding process) the applicator 102 so that it is encapsulated, and thus reducing the need for adhesives in the assembly. Where the coupling body is moulded in this way it may fit tightly around the applicator so that there is no clearance between them. In other embodiments, a potting compound or adhesive 115 may be provided between the applicator 102 and the coupling body 114. By encapsulating the applicator 102 within the coupling body 114 in this way a secure connection between the applicator 102 and the coupling body 114 may be formed.

The coupling body 114 further comprises a tubular member coupling interface 118 at which the coupling body 114 is coupled to the tubular member 112. The tubular member coupling interface 118 may be located at the proximal end of the coupling body 114. The tubular member coupling interface 118 may take a number of different forms, which may include an overlapping joint between the tubular member 112 and the coupling body 114 as will be described in more detail later. The profile and length of the interface may be optimised to enhance welding, for example providing a lap-joint, butt-joint or a lap-butt hybrid joint. Other types of coupling may however be used. The wall thickness of the overlapping section 118 of the coupling body 114 at the coupling interface 120 may be chosen or varied to create the optimal mechanical response. This could include creating a graded stiffness or strain relief effect between the applicator 102 and the tubular member 112.

The coupling body 114 further comprises a deformable member coupling interface 120 at which the coupling body 114 is coupled to the deformable member 110. The deformable member coupling interface 120 may take different forms depending on the materials used to form the deformable member 110 and the coupling body as will be described in more detail later. The coupling interface may, for example, comprise an adhesive or welded joint. The profile and length of the interface may be optimised to enhance welding, for example providing a lap-joint, butt-joint or a lap-butt hybrid joint. Other types of coupling may however be used.

The coupling body 114 further comprises a pointed distal tip 114a adapted for piercing tissue. The pointed distal tip of the coupling body 114 forms the distal tip of the ablation probe 100 and may have any suitable shape for piecing tissue. It may, for example, be a three sided trocar shape, conical shape or bevel cut. It may be formed by a moulding, grinding or ablation process.

The coupling body 114 acts to encapsulate the applicator 104 and provide a secure coupling to the deformable member 110 and the tubular member 112. The material from which the applicator is formed (e.g. ceramic) may be difficult to machine and bond to other components formed from different materials. By encapsulating the applicator a direct connection between the applicator and the deformable member and/or tubular member is not required. The indirect coupling via the coupling body may aid mechanical strength, while provide desired levels of flexibility and ease of manufacture by reducing complexity.

The portion of the coupling body surrounding the antenna may be suitably thin walled so as not to compromise the operation of the applicator. The portion of the coupling body surrounding the applicator may for example have a wall thickness of between 50 µm and 150 µm. In one embodiment it may be 80 µm. The dielectric permittivity and the relative wall thickness of the coupling body where it overlaps with the applicator are important to control power delivery performance.

A coupling interface 122 between the feeding cable 104 and the coupling body 114 may also be provided. This may aid the strength of the coupling between the applicator 102, feeding cable 104 and coupling body 114. The feeding cable coupling interface 122 may take the form of an adhesive (e.g. UV cure or cyanoacrylate adhesive) or potting compound (e.g. an epoxy) between an outer surface of the feeding cable 104, and the interior surface of the cavity 116. In other embodiments, other types of bonding interface between the feeding cable 104 and the coupling body 114 may be provided. In some embodiment, a mechanical lock may be formed between the components to ensure secure coupling. In yet further embodiments, no direct coupling may be provided between the feeding cable and the coupling body 114.

The coupling body 114 may be at least partly formed from a plastics material. This may provide a more favourable alternative material with which to bond the deformable member 110 and the tubular member 112 compared to the ceramic of the applicator 104. In some embodiments, all of the coupling body may be formed from a plastics material.

The coupling interfaces 118, 120, 122 between the coupling body 114 and the deformable member 110, tubular member 112, applicator 104 and feeding cable are shown as cross-hatched patterned regions in Figure 4. The types of bond used may be tailored to the material of the components being bonded and the desired level of strength and flexibility as described below. In some embodiment, a mechanical lock may be formed between the components to ensure secure coupling.

The material used to form the coupling body 114 may be selected to provide the optimum mechanical strength and flexibility, thermal resistance and suitability for processing. The material used to form the coupling body may have any one or more of the properties in the following paragraph:
For example, the material may be chosen with high mechanical toughness (e.g. Charpy notched impact strength in a range between 1 and 50 kJ/m² to ensure it can withstand high strains at the tubular member coupling interface 118. A material may be selected with high hardness (Rockwell Harness (m-Scale) in a range between 10 and 50) or flexural modulus (in a range between 1-50 GPa) to increase the sharpness and tissue piercing performance of the distal tip. The material may be selected based on its thermal resistance (e.g. a heat deflection temperature in a range between 80 and 400 degrees Celsius at 1.8 MPa) to ensure it can withstand heating effects originating from the applicator 104 during therapy delivery (which can exceed 100 degrees Celsius). The material may be selected based on its rheology characteristic and its suitability for moulding of geometries with thin wall sections (e.g. wall sections have a thickness of < 0.15mm, typically 0.08 mm).

The material chosen for the coupling body may provide an optimum combination of these properties. The coupling body may therefore be formed from glass filled (e.g. 30% glass filled) Nylon 6, Polyetheretherketone (PEEK), a Liquid Crystal Polymer (LCP), polycarbonate (PC), a thermoset material (e.g. polyimide), polystyrene, PET. Other suitable material may be used.

In one embodiment, at least part of the coupling body 114 may be formed from a transparent material such as a transparent plastics material (e.g. polycarbonate, polyethylene terephthalate (PET) or polystyrene). By forming the coupling body from a transparent material, the joint between the coupling body 114 and the components to which it is bonded may be visible after assembly. This may allow the use of UV curing adhesive to form bonds between components. For example, a region of the coupling body 114 forming any one or more of the coupling interfaces with the deformable member 110, tubular member 112, feeding cable 104 or which surrounds the applicator 102 may be formed from a transparent plastics material. In some embodiments, all of the coupling body 114 may be formed from a transparent plastics material.

In one embodiment, at least part of the coupling body may be formed from nylon or glass reinforced nylon. For example, a region of the coupling body 114 forming any one or more of the coupling interfaces with the deformable member 110, tubular member 112, feeding cable or which surrounds the applicator 104 may be formed from glass reinforced nylon. In some embodiments, all of the coupling body 114 may be formed from glass reinforced nylon.

In order to provide compatibility with the coupling body, the deformable member 110 may be formed from the same or similar material as the coupling body 114. The deformable member and at least a portion of the coupling body forming the deformable member coupling interface may, for example, both be formed from materials having compatible melting temperatures. By compatible, we mean suitable for forming a welded joint i.e. the melting temperature may be equal or similar (e.g. having a difference of 100 degrees (or 50 degrees) or less between them).

For example if the deformable member is formed of Nylon 6, the coupling body may be formed of Nylon 6 also, or glass reinforced Nylon 6. The deformable member coupling interface 120 may then be formed by a welded joint formed by melting of the material forming the coupling body and the deformable member. The welded joint may be formed by a re-flow, laser or ultrasonic welding process, or another suitable process. This may provide an improved joint between them. A similar welding process may be used to join the coupling body 114 and the tubular member 112 if the tubular member is formed from a polymeric material (e.g. Nylon, Pebax, PEEK or any other polymer material).

The coupling body 114 may be formed from a single integral piece. In other words, in may be a single component formed from a continuous piece of the same material. This may provide improved mechanical strength and aid manufacture. In other embodiments, different parts of the coupling body may be formed from different materials. For example, the region of the coupling body surrounding the applicator may be transparent or the region of the coupling body forming the deformable member coupling interface may be formed from glass reinforced nylon to allow a welded joint to be formed. In some embodiments, the portion of the coupling body forming the pointed tip may be formed from a different material to the rest of the coupling body. The pointed tip section maybe formed of a non-polymeric material (e.g. a metal or ceramic) which may have superior tissue piercing properties. In this embodiment the coupling body may include a geometry or interlocking shape to receive or engage with a separate component forming the pointed tip. This may allow the pointed tip component to be fixed securely to the coupling body (e.g. using an adhesive or insert moulding process).

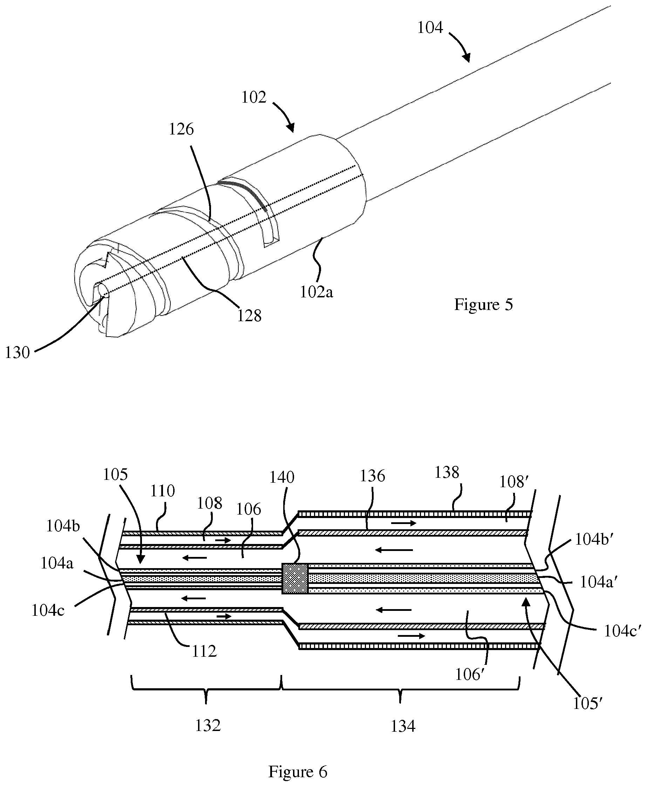

As can be seen illustrated in Figure 2, the applicator 102 comprises an antenna body 102a having a generally cylindrical shape. A close up of the antenna body 102a is also shown in Figure 5, in which only the applicator 102 and the connected part of the feeding cable 104 are shown. The applicator 102 may further comprise an antenna conductor 124. The antenna conductor 124 is formed from an electrically conducting element (e.g. a wire) that is electrically connected, or is part of, the feeding cable 104. In the presently described embodiment, the feeding cable 104 comprises an inner conductor 104a, an outer conductor 104b and a dielectric material between them. The antenna conductor 124 is formed from part of the inner conductor 104a of the feeding cable 104 that extends beyond the distal end of the dielectric material and outer conductor 104b. In other embodiments, a separate conducting wire may be coupled to the feeding cable 104 to form the antenna conductor.

Referring again to Figure 2 and particularly Figure 5, the antenna body 102a comprises an outer surface having a channel 126 in which the antenna conductor 122 is received. The channel may extend along a helical path around a central axis of the antenna body 102a to form a helical shaped antenna conductor 124. Other shapes of antenna conductor 122 may be provided by using a correspondingly shaped path around the outer surface of the antenna body. The channel in the antenna body 102a may help maintain the antenna conductor 124 in the desired shape. It may also allow the antenna conductor 124 to fit compactly within the cavity 116 of the coupling body 114. The antenna conductor 122 may, for example, be easier to manufacture compared to the use of conducting material deposited on the surface of the antenna to form the antenna conductor 122.

The antenna conductor 122 may extend through an axial through hole 128 formed in the antenna body 104a. The antenna conductor 128 may extend along the central axis of the applicator body 102a, through the applicator body, and out of a hole 130 at the distal end of the applicator body 102a. The antenna conductor 128 may then extend back along the length of the ablation probe so that it overlaps the portion of its length within the antenna body. This may allow the antenna conductor 124 to form a secure connection with the applicator body 102a. This arrangement may also aid manufacture. For example, the antenna conductor 124 may be provided with a convenient route into and out of the antenna body without requiring a complex passage machined through the antenna body 102a.

Referring again to Figures 3 and 4, the coupling interface 118 at which the coupling body 114 is coupled to the tubular member comprises an overlapping joint (e.g. a lap joint). The joint is formed from an overlapping portion 118a of the coupling body 114 that extends within the tubular member 112 so that they overlap one another. A bond interface is formed between an outer surface of the overlapping portion 118a of the coupling body 114 and the inner surface of the tubular member 112. This may allow a large connection area and so a secure bond between them. The overlapping portion 118a of the coupling body 114 may have a reduced thickness to form a half lap joint. This may reduce the overall size of the joint between the two components. The bond between the coupling body and the tubular member 112 may be formed by adhesive on the contact surface between the two components. In other embodiments, a welded joint may be used. In the described embodiment, the tubular member 112 fits around the outside of the overlapping portion 118a. In other embodiments the reverse may be the case, with the overlapping portion 118a of the coupling body extending around the tubular member 112. The overlapping portion may help to prevent kinking at the interface between relatively non-flexible applicator and relatively flexible parts of the ablation probe i.e. the tubular member and the feeding cable.

The thickness of the overlapping portion may be in a range between 50 µm and 150 µm. In one embodiment it may be 80 µm. This may provide the desired level of flexibility. In one embodiment, the thickness of the overlapping portion may vary along its length to provide improved control of the stiffness.

Referring again to Figure 1, the ablation probe 100 generally comprises two portions: a needle portion 132 and a catheter portion 134. The needle portion 132 may be arranged at the distal end of the ablation probe 100 and is adapted to be inserted into tissue during use to reach the desired ablation location. The catheter portion 134 may be provided at the proximal end of the ablation probe 100 and is arranged to supply electromagnetic energy and a flow of coolant to and from the needle portion 132. The catheter portion 134 may have an extended length and flexibility for endoscopic use. In other embodiments, a shorter, more rigid catheter portion 134 may be provided for percutaneous use.

In some embodiments, the needle portion 132 may form a small part of the overall length of the ablation probe. For example, the needle portion may be 5 mm to 2000 mm in length, and preferably may be around 70 mm in length. The length of the needle portion 132 may be chosen according to the anatomy to be accessed. For example, the needle portion may be approximately between 10 and 100 mm long for delivery of therapy to organs including the pancreas, or lung, or longer (for example 100-400mm in length) for delivery of therapy percutaneously. A longer length of needle portion may be more suitable for accessing parts of the lung, for example. The catheter portion may be around 1000 mm to 2000 mm in length, and preferably around 1400 mm in length. The length of the catheter portion may be chosen according to the position of the ablation site which must be reached.

In other embodiments, the needle portion 132 of the ablation probe (e.g. that having the deformable member) may form a greater proportion of the length of the ablation probe. In some embodiments, the entire length of the ablation probe may be formed by the needle portion 132 (i.e. such that a separately defined catheter portion is absent). In such an embodiment, the deformable member 110, which is provided in the needle portion 132, may extend along the majority or all of the length of the ablation probe. In such an embodiment, the catheter portion may not be required. For example, if the ablation probe is to be used percutaneously the catheter portion 134 may be shorter than for endoscopic use, or may not be required.

The junction between the needle portion 132 and the catheter portion 134 is shown in Figure 6. The needle portion 132 comprises the deformable member 110, the applicator 102, a distal portion 105 of the feeding cable, a distal portion of the coolant delivery path 106, and the coolant return path 108 (provided within the deformable member). The catheter portion 134 comprises a proximal portion 105' of the feeding cable, a proximal portion 106' of the coolant delivery path, and a catheter portion (or second) coolant return path 108'. The proximal portion of the coolant delivery path 106' is formed by a space between a first or inner catheter tube 136 housing the proximal portion 105' of the feeding cable and the proximal portion of the feeding cable 104. The second coolant return path 108' is formed by the space between the first catheter tube 136 and a surrounding second or outer catheter tube 138. Both of the first and second catheter tubes 136, 138 may be non-deformable, in contrast to the collapsible coolant delivery path provided within the needle portion 132. In other embodiments, any other suitable arrangement of channels or conduits may be provided to form the coolant return and coolant delivery paths within the catheter portion 134.

The greatest cross sectional size of the needle portion 132 may be less than the greatest cross sectional size of the catheter portion 134 (when the deformable member is in the insertion configuration). In other words, the cross section size (e.g. diameter) of the needle portion 132 at its largest point may be less that the cross sectional size (e.g. diameter) of the catheter portion 134 at its greatest point. This may allow the needle portion to access an ablation site whilst reducing any potential for tissue damage. The catheter portion on the other hand may be sized to fit through the working channel of the device with which it is used.

In the described embodiment, the feeding cable 104 is formed by two lengths of cable (the distal portion 105 and the proximal portion 105') joined at the boundary between the needle portion 132 and the catheter portion 134.

The feeding cable 104 may be formed by two lengths of coaxial cable to form an electrical circuit to deliver electromagnetic energy to the applicator 102. The distal portion 105 comprises the inner conductor 104a, outer conductor 104b and dielectric 104c already described. The proximal portion 105' similarly comprises an inner conductor 104a', an outer conductor 104b' and a dielectric 104c'.

In other embodiments a single feeding cable may be used having regions of different thickness to form the distal and proximal portions. In other embodiments, any other suitable conductor may be provided to deliver a supply of suitable electromagnetic energy to the applicator 102. The ablation probe 100 may further comprise a connector 140 arranged to mechanically and electrically connect the distal portion of the feeding cable 105 to the proximal portion of the feeding cable 105'. The connector 140 may connect the different portions of the feeding cable while maintaining an effective impedance match, minimising electrical losses and ensuring a compact configuration of the ablation probe 100.

In the described embodiment, the distal portion of the feeding cable 105 has a corresponding distal cross sectional size, and a proximal portion of the feeding cable 105' has a corresponding proximal cross sectional size, wherein the distal cross sectional size is less than the proximal cross sectional size. The size (e.g. diameter) of the conductor is therefore optimised based on its position within the ablation probe 100. The cross sectional sizes may be chosen to optimise (e.g. maximise) the feeding cable power handling, while also reducing electrical losses and optimising the mechanical strength of the ablation probe 100. In other words, the length of the smaller cross section portion of the feeding cable is minimised by connecting it to a larger cross section feeding cable (e.g. a more efficient cable) for the portion of the ablation probe 100 outside of the needle portion 132. This part of the ablation probe 100 does not need to be inserted into tissue so a small profile is not as important. The cross section of the feeding cable in the catheter portion 134 is therefore increased to reduce power loss where a small cross section is less important.

The needle portion of the ablation probe may therefore have a smaller overall cross sectional size compared to the catheter portion. The needle portion is therefore optimised for insertion into tissue, whilst the catheter portion is optimised for power delivery over the long length of a device working channel through which it is inserted. In use, only the needle portion may protrude from the working channel through which the ablation probe is inserted. It is therefore important for the needle portion to have a relatively small cross sectional size to reduce tissue damage. For the catheter portion a relatively larger cross sectional size can be used. Compared to the needle portion, the catheter portion is instead optimised for power delivery along the length of the working channel. In one example, the needle portion, when the deformable member is in the insertion configuration, may have an overall diameter of 1 mm at its largest point. The catheter portion may have an overall diameter of less than 2 mm at its largest point.

In other embodiments, the cross sectional size of the distal and proximal portions of the feeding cable may be the same. In this case, a reduction in overall size of the needle portion compared to the catheter portion may still be provided by the use of the deformable member.

In the described embodiment, the deformable member 110 extends along at least part of the length of the needle portion 132 as shown in the Figures. The deformable member 110 may, for example, extend from at or near the boundary between the needle portion 132 and the catheter portion 134 and end at or near the distal end of the applicator 102 (e.g. where it is coupled by the deformable member coupling interface 120). The coolant may therefore flow through the deformable member 110 along the length of the ablation probe (e.g. a flow of coolant may be provided between an inlet and an outlet of the deformable member, the inlet and outlet being spaced apart along the length of the ablation probe). The deformable member 110 may be fluidly connected to the non-deformable second catheter tube 138 at a boundary between the needle portion 132 and the catheter portion 134. The coolant may therefore flow through the deformable member 110 (when in the deployed configuration) and then through the non-deformable outer catheter tube 138 in the catheter portion 134 to reach the proximal end of the ablation probe 100.

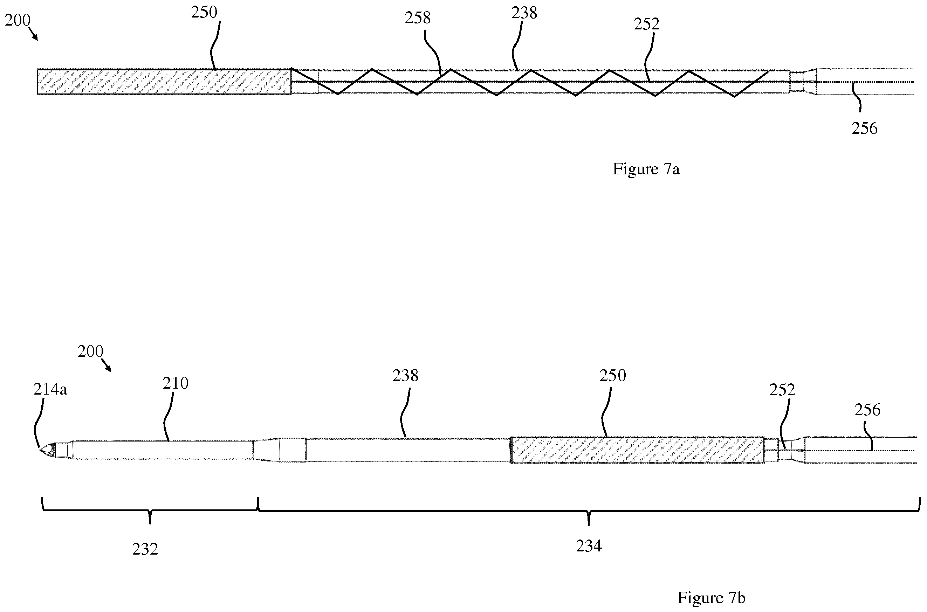

Another embodiment of an ablation probe 200 is shown in Figures 7a to 9b. The ablation probe illustrated in Figures 7a to 9b has corresponding features to the embodiments already described. Corresponding reference numbers have been used accordingly. The ablation probe 200 comprises a needle portion 232 and a catheter portion 234. As described above, the needle portion 232 comprises a deformable member 210, applicator, a distal portion of the feeding cable, a distal portion of the coolant delivery path, and the coolant return path (provided within the deformable member). The catheter portion comprises a proximal portion of the feeding cable, a proximal portion of the coolant delivery path, and a catheter (or second) coolant return path. The proximal portion of the coolant delivery path is formed by a space between a first or inner catheter tube housing the proximal portion of the feeding cable and the feeding cable. The catheter coolant return path is formed by the space between the first catheter tube and a surrounding second or outer catheter tube 238.

The ablation probe 200 comprises a sheath member 250. The sheath member 250 is movable between a first position (i.e. a sheathed position) in which it surrounds the pointed tip 214a of the coupling body 214 and a second position (i.e. an unsheathed position) in which the pointed tip 214a is uncovered or exposed by the sheath member 250. Figure 7a shows the sheath member 250 in the first position, with the second position illustrated in Figure 7b. The sheath member 250 may move the length of the ablation probe between the first and second positions. A movement in a proximal direction provides movement from the first to the second position, with distal movement providing movement from the second to the first positions. The sheath may therefore overlap the sharp tip of the ablation probe during delivery of the device through the working channel of an endoscope or similar device. This may prevent damage to the inner surface of the endoscope (or navigation system) working channel caused by the sharp tip. By moving the sheath member 250 to the second position it may be exposed after the pointed tip 214a has exited the working channel (e.g. of the ENB system) to enable the device to pierce into the target lesion.

The sheath member 250 is formed from a tubular member that fits around or surrounds the body of the ablation probe. The sheath member may form an outer layer of the ablation probe so as to form an outer protective barrier between the ablation probe and the working channel of a delivery device with which it is used. The sheath member 250 may be formed from a material suitable to withstand piercing by the pointed tip 214a, but still have the desired flexibility to allow delivery of the ablation probe along a tortuous route. The sheath member 150 may, for example, be formed from a plastics material (e.g. Pebax or Nylon) or a braid / coil reinforced polymer tube.

As can be seen in Figure 7a and 7b the sheath member 250 extends only part way along the length of the ablation probe 200 between its distal and proximal ends. The ablation probe 200 further comprises one or more fixing wires or control lines 252 connected to the sheath member 250. The control lines may be elongate wires or cables running the length of the ablation probe. They may, for example, be described as pull wires. The fixing wires extend along the length of the ablation probe between the sheath member 250 and a position at or near to the proximal end of the ablation probe 200. The fixing wires are adapted to allow control of the position of the sheath member 250 and facilitate movement between the sheathed and unsheathed positions. The fixing wires may be connected to a portion of the handle that connects directly to endoscope or bronchoscope, ENB system or other delivery system. This ensures it cannot move distally relative to the delivery system. The handle may have two parts that are movable relative to each other: a first part may be coupled to the ablation probe and the second part coupled to the delivery system (fixed relative to the working channel) The ablation probe 200 is connected to the moveable element (i.e. first part) of the handle allowing it to be advance forward relative to the sheath, into the target lesion.

The fixing wires 252 may be formed by any suitable elongate members. They may, for example, be formed from metal or plastic wires. The control lines may be formed from rods or similar structures along some or all of their length. The rods may be configured not to buckle if handle system is used to move the sheath 250 distally over the applicator 102.

By providing a sheath member 250 that extends over only part of the length of the ablation probe the space that it takes up may be reduced. This allows a more compact arrangement, which may be particularly advantageous when the ablation probe is used with an endoscope or similar device where space inside the working channel is limited. Part of the length of the sheath member 250 is effectively replaced by the fixing wires 252, rather than extending the sheath all of the way to the proximal end of the device to allow it to be positioned during use. In this way, a more compact ablation probe may be provided. More space may be allowed for the coolant flow paths and feeding cable within the size constraint of the working channel in which it is to be inserted.

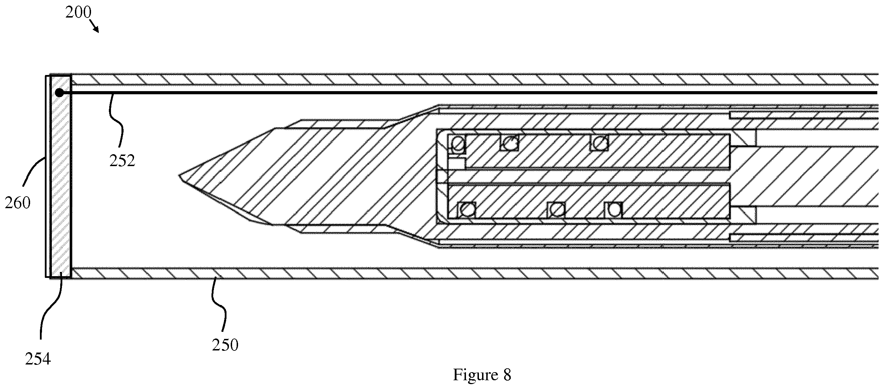

The one or fixing wires 252 may be connected at or near the distal end of the sheath member 250. This may provide improved control of the movement of the sheath member 250. In one embodiment, the sheath member 250 may comprise a reinforcing ring 254 at its distal end. An example of such an embodiment is shown in Figure 8. In this embodiment, the one or more fixing wires 252 are connected to the reinforcing ring 254. In some embodiments, a plurality of fixing wires may be provided. The plurality of fixing wires may be distributed evenly around the sheath member 152 (e.g. equally around its circumference, or equally around the circumference of the reinforcing ring) so as to apply an even force.

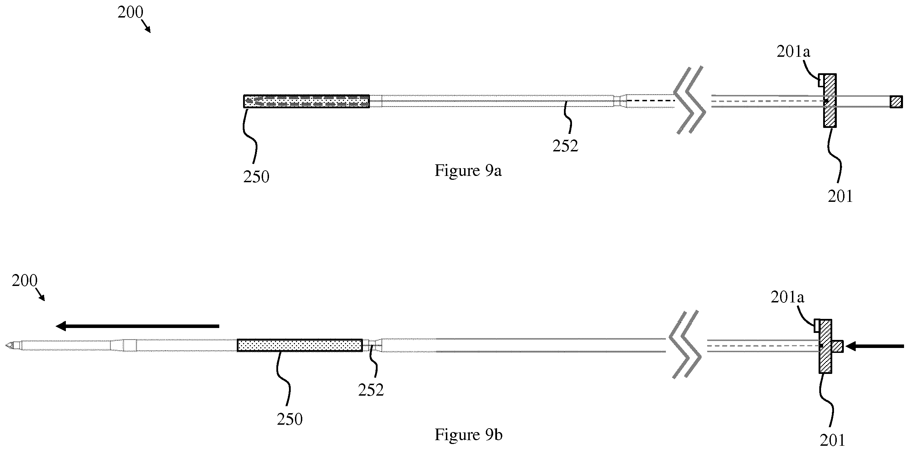

Referring to Figures 9a and 9b the ablation probe is coupled to a handle 201 at or near its proximal end. The ablation probe 200 and handle 201 may together form an ablation probe assembly. The handle 201 is adapted to allow manipulation of the ablation probe during use. The ablation probe is slidably coupled to the handle 201 so that it can be moved relative to the handle between an extended and retracted position (shown by the arrows in Figure 9b). The retracted position is shown in Figure 9a, with the extended position shown in Figure 9b. The handle portion 102 comprises a connecting mechanism 201a with which the handle 201 is connectable to a delivery device (e.g. an endoscope) with which the ablation probe 200 is to be used. When connected, the handle 201 may be fixed relative to the working channel of the delivery device. Once fixed in this may, sliding movement of the ablation probe 200 relative to the handle 201 causes sliding movement of the ablation probe 200 along the length of the working channel. This may cause the distal end of the ablation probe 200 to extend from the distal end of the working channel to access tissue when the delivery device has been positioned at the required location within the body.

In the present embodiment, the one or more fixing wires 252 are connected between the handle 201 and the sheath member 250. The fixing wires are arranged to restrict the range of movement of the sheath member 250 in a proximal direction away from the handle 201. The sheath member 250 is therefore restricted from moving along the length of the ablation probe 200 in a proximal direction away from the handle 201 by a maximum distance set by the length of the fixing wires 252. The fixing wires may be substantially inelastic so that they can pull back the sheath. This means that movement of the ablation probe relative to the handle 201 causes movement of the sheath member 250 from the sheathed to the unsheathed configuration. This may allow the ablation probe to be unsheathed using the same action that extends its distal end from the working channel, thus facilitating ease of use.

As already described, the one or more fixing wires 252 extend along the length of the ablation probe. In the described embodiment, the fixing wires 252 extend within a channel 256 or lumen formed in the outer catheter tube of the ablation probe. This may reduce the space taken up by the fixing wires and allow a compact arrangement. The channel or lumen housing the control lines 252 may be formed in the wall of the catheter tube, or in a protrusion on the outer surface of the tube.

The sheath member 250 may form a friction fit with the body of the ablation probe which it surrounds. For example, a friction fit may be formed with the deformable member 210 or any section of the catheter (e.g. any other suitable outer part of the ablation probe away from the deformable member). The friction fit may act to retain the sheath member 250 in the sheathed position during delivery through a tortuous path, until it is retracted using the fixing wires. This may help to keep the sheath member 250 in the sheathed position during insertion into the working channel.

The ablation probe may comprise a biasing member 258 arranged to bias the sheath member 250 towards the sheathed position. The biasing member 258 may bias the sheath member 250 towards distal direction along the length of the ablation probe 200. The biasing member 258 may be a coiled spring arranged around the body of the ablation probe. Other types of biasing member may however be used. The biasing member may be provided in addition or alternatively to the friction fit. The stiffness of the biasing member 258 may be optimised to ensure ease of use. It shall ensure the sharp tip is re-sheathed when withdrawn from the lesion (i.e. provide adequate force to move the sheath from the un-sheathed to sheathed position when required).

The sheath member may comprise a covering member 260 arranged to cover the pointed tip when the sheath member is in the first position. The covering member is adapted to be pierced (i.e. it is frangible) by the pointed tip when the sheath member is moved from the first position to the second position to expose the pointed tip. The covering member may be formed from a membrane covering the distal end of the sheath member as shown in Figure 8. The membrane is pierced by the pointed tip of the ablation probe only when it is exposed from the sheath member. The membrane is pierced by the pointed tip when the control lines pull back and the sheath member slides from the sheathed to the unsheathed positions. The covering member may help to keep the sheath member in place when it travels along the working channel. When the control lines apply force to the sheath, the pointed tip pierces the membrane and the sheath slides to uncover the pointed tip.

In the embodiment shown in Figures 7a to 9b the sheath member 250 is used in combination with the ablation probe described with reference to Figures 1 to 6. The sheath member may however be used in combination with other ablation probes, for example those in which the deformable member and/or the coupling body may be absent.

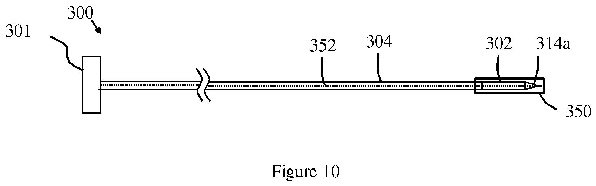

The sheath member may, for example, be used with any ablation probe having an applicator arranged to apply radiation to heat surrounding tissue; a feeding cable arranged to supply electromagnetic energy to the applicator; and a pointed distal tip adapted for piercing tissue. An example of such an embodiment is shown schematically in Figure 10, which illustrates an ablation probe 300 comprising an applicator 302. The applicator 302 is arranged to apply radiation to heat surrounding tissue as described in connection with other embodiments. The ablation probe 300 comprises a feeding cable 304 arranged to supply electromagnetic energy to the applicator 302. A distal end of the ablation probe comprises a pointed distal tip 314a adapted for piercing tissue. The pointed tip may be provided on the component forming the distal end of the ablation probe, which may be a coupling body as already described, or may be a pointed tip of the applicator or separate tip component. The ablation probe further comprises a sheath member 350 movable between a first position in which it surrounds the pointed tip and a second position in which the pointed tip 314a is uncovered. The sheath member 350 extends part way along the length of the ablation probe between the ablation probe's distal and proximal ends. The ablation probe comprises one or more fixing wires 352 connected to the sheath member. The fixing wires extend along the length of the ablation probe between the sheath member and a position at or near the proximal end of the ablation probe.

The ablation probe 300 may be coupled to a handle 301 as described above in connection with Figures 6a to 9b. The fixing wires 352 may be connected between the sheath member 352 and the handle 301 so that movement relative to the handle causes movement of the sheath member between the sheathed and unsheathed positions. Any of the features described in connection with the embodiments of Figures 6a to 9b can be provided in the embodiment of Figure 10.

Various modifications will be apparent to the skilled person without departing form the scope of the claims. Any feature disclosed in connection with one embodiment may be used in combination with the features of another embodiment.

## Claims

1. An ablation probe, comprising:
an applicator arranged to apply radiation to heat surrounding tissue;
a feeding cable arranged to supply electromagnetic energy to the applicator;
a coolant flow path forming a coolant supply circuit;
a tubular member housing at least part of the feeding cable, wherein a part of the coolant flow path is defined by a space between the feeding cable and the tubular member;
and a coupling body, the coupling body comprising:
a cavity in which the applicator is at least partly encapsulated;
a coupling interface at which the coupling body is coupled to the tubular member;
and a pointed distal tip adapted for piercing tissue.

2. An ablation probe according to claim 1, further comprising a deformable member surrounding at least part of the tubular member, the deformable member being arranged to move between an insertion configuration and a deployed configuration, and wherein a part of the coolant path is defined between the deformable member and the tubular member when the deformable member is in the deployed configuration, and wherein the coupling body comprises a coupling interface at which the coupling body is coupled to the deformable member.

3. An ablation probe according to claim 1 or claim 2, wherein coupling body is formed at least partly from a plastics material.

4. An ablation probe according to any preceding claim, wherein any one or more of:
a) the coupling body is formed at least partly from a material having a Charpy notched impact strength in the range between 1 and 50 kJ/m²;
b) the coupling body is formed at least partly from a material having a Rockwell Harness (m-Scale) in the range between 10 and 50;
c) the coupling body is formed at least partly from a material having a flexural modulus in the range between 1-50 GPa;
d) the coupling body is formed at least partly from a material having a heat deflection temperature in the range between 80 and 400 degrees Celsius at 1.8 MPa;
e) the coupling body may be formed at least partly from a transparent material; and
f) when dependent on claim 2, the deformable member and at least a portion of the coupling body forming the coupling interface at which the coupling body is coupled to the deformable member are both formed from materials compatible for thermal welding.

5. An ablation probe according to any preceding claim, wherein:
the coupling body is formed at least partly from any one of: polycarbonate (PC), Polyetheretherketone (PEEK), nylon, glass reinforced nylon, Liquid Crystal Polymer (LCP), polystyrene, polyethylene terephthalate (PET) or polyimide.

6. An ablation probe according to any claim 5, wherein either or both of:
a) wherein the coupling body is formed from nylon or glass reinforced nylon and the tubular member is formed at least partly from a polymeric material such as Nylon, Pebax, polyethylene terephthalate (PET) or PEEK; and/or
b) when dependent on claim 2, wherein the coupling body is formed from nylon or glass reinforced nylon and the deformable member is formed at least partly from nylon.

7. An ablation probe according to any preceding claim, wherein the applicator comprises an antenna body and an antenna conductor, wherein the antenna body comprises an outer surface having a channel in which the antenna conductor is received, and optionally wherein the channel extends along a helical path around a central axis of the antenna body.

8. An ablation probe according to claim 7, wherein the feeding cable comprises an inner conductor, an outer conductor and a dielectric material between them, and wherein the antenna conductor is formed from part of the inner conductor of the feeding cable,
and optionally wherein part of the length of the antenna conductor extends in a distal direction from a distal end of the outer conductor and within an axial through hole formed in the antenna body.

9. An ablation probe according to any preceding claim, wherein a potting compound or adhesive is provided between the applicator and the coupling body.

10. An ablation probe according to any preceding claim, wherein either or both of:
a) the coupling body is insert moulded around the applicator; and/or
b) the coupling interface at which the coupling body is coupled to the tubular member comprises an overlapping portion of the coupling body that extends within or around the tubular member so that they overlap one another.

11. An ablation probe according to any preceding claim, further comprising a sheath member movable between a first position in which it surrounds the pointed tip of the coupling body and a second position in which the pointed tip is uncovered.

12. An ablation probe according to claim 11, wherein the sheath member extends part way along the length of the ablation probe between the distal and proximal ends of the ablation probe, and wherein the ablation probe comprises one or more control lines connected to the sheath member, the control lines extending along the length of the ablation probe between the sheath member and a position at or near the proximal end of the ablation probe,
and optionally the one or more control lines are connected at or near a distal end of the sheath member,
and further optionally, the sheath member is formed from a tube have a reinforcing ring at its distal end, wherein the one or more control lines are connected to the reinforcing ring.

13. An ablation probe according to claim 12, wherein the ablation probe is slidably coupled to a handle provided at or near its proximal end, and wherein the one or more control lines are connected between the handle and the sheath member, and the control lines are arranged to restrict the range of movement of the sheath in a distal direction away from the handle.

14. An ablation probe according to any of claims 11 to 13, wherein any one or more of:
a) when dependent on claim 12, the ablation probe comprises an outer catheter tube in which part of the coolant flow path is contained, and wherein the one or more control lines extend within a channel or lumen formed in the outer catheter tube;
b) the sheath member forms a friction fit with the body of the ablation probe around which it extends and/or comprises a biasing member arranged to bias the sheath member in a distal direction; and/or
c) the sheath member comprises a covering member arranged to cover the pointed tip when the sheath member is in the first position, the covering member being pierced by the pointed tip when the sheath member is moved from the first position to the second position to expose the pointed tip.

15. An ablation probe, comprising:
an applicator arranged to apply radiation to heat surrounding tissue;
a feeding cable arranged to supply electromagnetic energy to the applicator;
a pointed distal tip adapted for piercing tissue;
a sheath member movable between a first position in which it surrounds the pointed tip and a second position in which the pointed tip is uncovered, sheath member extending part way along the length of the ablation probe the distal and proximal ends of the ablation probe; and
one or more control lines connected to the sheath member, the control lines extending along the length of the ablation probe between the sheath member and a position at or near the proximal end of the ablation probe.
